# EUROPÄISCHE PATENTANMELDUNG

(11) **EP 4 403 130 A1**
(43) Veröffentlichungstag der Anmeldung: **24.07.2024**
(21) Anmeldenummer: 23152046.1
(22) Anmeldetag: 17.01.2023
(51) Int. Cl.: A61B 90/50

(54) **FEDERARMVORRICHTUNG UND TRAGARMSYSTEM ZUM HALTEN EINES MEDIZINISCHEN GERÄTS**

(71) Anmelder: Ondal Medical Systems GmbH, 36088 Hünfeld (DE)
(72) Erfinder: Muratidi, Georg, 36280 Oberaula (DE)
(74) Vertreter: Graf von Stosch Patentanwaltsgesellschaft mbH

(57) **Zusammenfassung**

Die vorliegende Erfindung betrifft eine Federarmvorrichtung (1) für ein Tragarmsystem (100) zum Halten eines medizinischen Geräts, umfassend ein Federrohr (3) und eine zumindest teilweise im Inneren des Federrohres (3) angeordnete, in Längsrichtung (2) des Federrohres (3) relativ zum Federrohr (3) verschiebbare Federstange (4). Die Federarmvorrichtung ist weiterhin durch eine Klemmeinheit (30) gekennzeichnet. Die Klemmeinheit ist zum lösbaren Aufbringen einer Klemmkraft auf die Federstange (4) am Federrohr (3) in Längsrichtung (2) betrachtet ortsfest angeordnet. Weiterhin wird ein Tragarmsystem (100) zum Halten zumindest eines medizinischen Geräts, umfassend zumindest eine Federarmvorrichtung (1) offenbart.

## Beschreibung

### Technisches Gebiet

Die vorliegende Erfindung betrifft eine Federarmvorrichtung für ein Tragarmsystem zum Halten eines medizinischen Geräts und ein Tragarmsystem zum Halten eines medizinischen Geräts.

### Stand der Technik

Tragarmsysteme werden beispielsweise in Operationssälen zum örtlichen verlagerbaren Halten einer medizintechnischen Einrichtung bzw. eines medizintechnischen Geräts genutzt. Beispielsweise kann dazu ein Tragarm mittels einer Lageranordnung beweglich an einer Decke, einer Wand oder einem Boden verankert sein. Auch eine Befestigung auf einem Gestell mit Rollen ist möglich. Ein Tragarmsystem, welches zum Halten medizinischer Geräte ausgebildet ist, kann auch als medizinisches Tragsystem bezeichnet werden. Tragarmsystem weisen üblicherweise wenigstens eine Bewegungsachse auf, damit das gehaltene medizinischen Gerät, welches auch als medizintechnisches Gerät bezeichnet werden und beispielsweise eine OP-Lampe, einen Monitor, Untersuchungsgeräte, einen oder mehrere Injektoren, OP-Besteck oder zahnmedizinische Bohrer aufweisen kann, gemäß jeweiliger Anforderungen bewegt werden kann. Die Lagerung, welche die Bewegung ermöglicht, kann auch als Gelenkvorrichtung oder Tragarmgelenkvorrichtung bezeichnet werden, wobei diese auch einen Tragarm umfassen kann. Damit kann das medizinische Gerät in eine angenehme und sichere Position zum Durchführen jeweiliger medizinischer Prozeduren und Überwachungen verbracht werden. Das örtliche Verlagern medizinischer Geräte ist bei vielen medizinischen Prozeduren essentiell, um dem medizinischen Personal die Arbeit zu erleichtern oder überhaupt zu ermöglichen.

Durch ein Schwenken eines Tragarms kann jedoch ein Hebelarm bezüglich dessen Lagerung verändert werden. Insbesondere bei einem Schwenken des Tragarms um eine im Wesentlichen horizontale Achse, wodurch die Höhe des daran gehaltenen medizinischen Geräts verändert werden kann, wird ein Hebelarm der Gewichtskraft des medizinischen Geräts gegenüber der Befestigung bzw. Lagerung des Tragarms verändert. Dennoch ist es gewünscht, dass das medizinische Gerät in einer durch das Schwenken des Tragarms eingestellten Position verbleibt und eine Verstellung der Position des medizinischen Geräts, insbesondere entgegen der Schwerkraft, ohne exzessiven Kraftaufwand für einen Bediener möglich sein soll.

Zu diesem Zweck werden häufig federbalancierte Tragarme, vorliegend als Federarme bzw. Federarmvorrichtungen bezeichnet, genutzt. Dabei werden zwei relativ zueinander bewegbare Armelemente vorgesehen, welche sich mittels einer Feder gegenseitig stützen. Durch Verschwenken des Federarms ändert sich der Hebelarm der Federkraft korrespondierend zu dem Hebelarm der Traglast, wodurch das medizinische Gerät in unterschiedlichen Positionen gleichermaßen gut gehalten werden kann. Alternativ oder zusätzlich kann auch die Feder durch das Schwenken komprimiert oder gestreckt werden, wodurch ebenfalls eine korrespondierende Stützkraft geändert werden kann.

Derartige Tragarmgelenkvorrichtungen bzw. Federarmvorrichtungen sind beispielsweise der WO 2016/074787 A1 oder der EP 3 861 958 A1 zu entnehmen.

### Darstellung der Erfindung

Ausgehend von dem bekannten Stand der Technik ist es eine Aufgabe der vorliegenden Erfindung, eine verbesserte Federarmvorrichtung für ein Tragarmsystem zum Halten eines medizinischen Geräts, sowie ein entsprechendes Tragarmsystem zum Halten eines medizinischen Geräts bereitzustellen. Insbesondere soll ein kostengünstiges Halten von medizinischen Geräten dauerhaft in einer vorgegebenen Stellung einfach ermöglicht werden.

Die Aufgabe wird durch eine Vorrichtung für ein Tragarmsystem zum Halten eines medizinischen Geräts mit den Merkmalen des Anspruchs 1 gelöst. Vorteilhafte Weiterbildungen ergeben sich aus den Unteransprüchen, der Beschreibung und den Figuren.

Entsprechend wird eine Federarmvorrichtung für ein Tragarmsystem zum Halten eines medizinischen Geräts vorgeschlagen, umfassend ein Federrohr und eine zumindest teilweise im Inneren des Federrohres angeordnete, in Längsrichtung des Federrohres relativ zum Federrohr verschiebbare Federstange.

Die Federarmvorrichtung umfasst ferner eine Klemmeinheit zum lösbaren Aufbringen einer Klemmkraft auf die Federstange, welche in Bezug auf die Längsrichtung des Federrohres ortsfest am Federrohr angeordnet ist. Durch das Aufbringen der Klemmkraft via der Klemmeinheit kann ein Verschieben der Federstange relativ zur Klemmeinheit in Längsrichtung und entsprechend zum ortsfest mit der Klemmeinheit verbundenen Federrohr unterbunden werden. Mit anderen Worten kann durch die Klemmeinheit ein Arretieren der Federstange relativ zum Federrohr erzielt werden. Die Federstange ist durch die Klemmeinheit, wenn sich diese in einem die Klemmkraft aufbringenden Zustand befindet, relativ zum Federrohr in einer festen Position fixiert.

Befindet sich die Klemmeinheit im gelösten Zustand, in welchem die Klemmeinheit keine Klemmkraft auf die Federstange ausübt, so kann die Federstange relativ zum Federrohr verschoben werden.

Aufgrund der Klemmeinheit kann insbesondere eine stufenlose Arretierung der Position der Federstange relativ zum Federrohr bereitgestellt werden. Denn beispielsweise im Gegensatz zu herkömmlichen Vorrichtungen mit einer vorgegebenen Zahl von voneinander beabstandeten festen Arretierpositionen kann via der vorgeschlagenen Klemmeinheit die Klemmkraft im Wesentlichen in jeder Position der Federstange relativ zum Federrohr aufgebracht werden und so der Federarm in im Wesentlichen jeder Position fixiert werden. Die stufenlose Arretierung ist zumindest über einen vorgegebenen Bewegungsumfang des Federrohres relativ zum Federrohr in Längsrichtung des Federrohres, mit anderen Worten in Richtung der Längsachse des Federrohres bereitgestellt.

Entsprechend ermöglicht die vorliegende Federarmvorrichtung ein präzises und sicheres Halten eines Federarms eines Tragarmsystems in einer festen Position, wobei die Federarmvorrichtung zugleich einen vergleichsweise einfachen Aufbau aufweist.

Die Möglichkeit, die Federarmvorrichtung in einer vorgegebenen festen Position zu fixieren (also zu arretieren), kann insbesondere bei Anwendungen vorteilhaft sein, bei welchen sich eine Belastung des die Federarmvorrichtung umfassenden Tragarmsystems ändert.

Beispielsweise kann es bei einem Halten eines medizinischen Injektors durch das Entleeren der Flüssigkeit aus dem Injektor über die Zeit zu einer Reduktion der durch einen Federarm bzw. eine Federarmvorrichtung gehaltene angehängte Traglast kommen. Durch diese Reduzierung kann es vorkommen, dass die auf den Federarm wirkende Vorspannung eines Vorspannelements der Federarmvorrichtung und die Traglast nicht mehr im Gleichgewicht sind. Als Folge könnte sich der Federarm aus seiner vorgesehenen Lage verschieben und dadurch den Injektor nach oben bewegen. Durch die vorgeschlagene Klemmeinheit kann die Position des Federarms bzw. der Federarmvorrichtung fixiert werden, so dass der Injektor stets an der vorgesehenen Position gehalten ist und eine sichere Behandlung eines Patienten erfolgen kann.

Analog zum Vorstehenden kann es bei als Dokumentationsterminal ausgebildeten medizinischen Geräten, bei welchen ein Monitor mit Tastatur angebunden ist, zu einer temporären Erhöhung der Traglast kommen, wenn bei der Bedienung der Tastatur zusätzlich eine Bedienkraft, verursacht durch das Tippen an der Tastatur, in das Tragarmsystem eingebracht wird. Durch die erfindungsgemäße Lösung kann die Federarmvorrichtung in einer vorgegebenen festen Position arretiert und gegen ein Absenken aufgrund der einwirkenden Bedienkraft verhindert werden.

Ferner kann durch die Arretierung der Federarmvorrichtung via Aufbringen der Klemmkraft durch die Klemmeinheit vermindert oder gar gänzlich verhindert werden, dass es aufgrund von während des Betriebs des durch das Tragarmsystem gehaltenen medizinischen Geräts auftretenden Schwingungen zu einem Wandern bzw. Driften der Federarmvorrichtung, genauer einem vibrationsbedingten kontinuierlichen oder diskontinuierlichen Verschieben der Federstange relativ zum Federrohr, kommt.

Der grundsätzliche Aufbau einer Federarmvorrichtung kann ferner der WO 2016/074787 A1, darin insbesondere Seite 14, Zeile 14 bis Seite 20, Zeile 20, und der EP 3 861 958 A1, darin insbesondere Absätze 48 bis 59 zu entnehmen, welche hierin durch Bezugnahme eingeschlossen sind.

Die Klemmeinheit ist vorzugsweise derart ausgebildet, dass durch das Aufbringen der Klemmkraft auf die Federstange die Federstange in ihrer Position relativ zum Federrohr arretiert ist.

Die Klemmeinheit weist vorzugsweise eine Kontaktfläche zum Aufbringen der Klemmkraft auf einen Außenumfangsabschnitt der Federstange auf, wobei die Klemmeinheit bevorzugt derart ausgebildet ist, dass im Zustand des Aufbringens der Klemmkraft die Kontaktfläche mit einer vorgegebenen Anpresskraft an den Außenumfangsabschnitt gepresst ist. Dadurch kann eine besonders sichere Klemmung vorgegebener Höhe erzielt werden.

Gemäß einer Ausführungsform kann die Klemmeinheit zumindest ein Klemmstück, bevorzugt zumindest eine Klemmbacke, und/oder einen Klemmring umfassen, wobei das zumindest eine Klemmstück und/oder der Klemmring bevorzugt die Kontaktfläche umfasst.

Der Klemmring umfasst vorzugsweise einen ringförmigen Grundkörper mit zumindest einem radial verlaufenden Schlitz. Mit anderen Worten ist der Klemmring ein Ringsegment mit sich gegenüberliegenden freien Enden als Begrenzung der Breite des Schlitzes in Umfangsrichtung des Ringsegments. Die gegenüberliegenden freien Enden sind bevorzugt in einem unbelasteten Zustand mit einem vorgegebenen Abstand voneinander beabstandet.

Die radiale Innenfläche des Klemmrings stellt die Kontaktfläche dar. Werden die freien Enden des Klemmrings aus dem unverspannten Zustand aufeinander zu gedrückt, so verringert sich entsprechend der Teilkreisdurchmesser, auf welcher die radiale Innenfläche vorliegt, so dass die durch den Klemmring geführte Federstange geklemmt werden kann.

Vorzugsweise umfasst die Klemmeinheit eine Betätigungseinheit zum Aufbringen und Lösen der Klemmkraft. Diese umfasst bevorzugt einen Klemmhebel oder einen Exzenterhebel.

Alternativ oder zusätzlich kann die Betätigungseinheit einen manuell betätigbaren Griff, bevorzugt einen Hebelgriff, einen Schraubgriff oder einen Sterngriff, umfassen.

Gemäß einer optimalen Ausführungsform umfasst die Federstange eine Gewindestange, welche zumindest an einem Abschnitt ein Gewinde umfasst. Die Federstange, bevorzugt deren Gewindestange, kann einen den Außenumfangsabschnitt bereitstellenden Zylinderflächenabschnitt aufweisen. Alternativ oder zusätzlich kann die Federstange auch eine bevorzugt auf ein Gewinde der Gewindestange aufgeschraubte Hülse umfassen, die den Außenumfangsabschnitt bereitstellt. Vorzugsweise ist die Hülse drehfest an der Federstange, bevorzugt an der Gewindestange, angeordnet, wobei bevorzugt die Hülse mittels eines Sicherungselements, vorzugsweise einem Sicherungsring, einem Sicherungsstift oder einer Sicherungsschraube, an der Federstange, bevorzugt an der Gewindestange, in ihrer Position relativ zur Federstange, bevorzugt zur Gewindestange, fixiert ist. Alternativ oder zusätzlich kann die Hülse auf die Federstange bzw. die Federstange, bevorzugt die Gewindestange, aufgepresst sein. Alternativ oder zusätzlich können die Gewindestange und die Hülse in einem verspannten Zustand verschraubt vorliegen. Ferner alternativ oder zusätzlich kann die Verschraubung von Gewindestange und Hülse selbsthemmend ausgebildet sein.

Der Außenumfangsabschnitt kann ferner ein Abschnitt eines Gewindes der Gewindestange sein.

Die Federstange ist vorzugsweise im Federrohr translatorisch gelagert, bevorzugt gleitgelagert, wobei bevorzugt die Federstange zumindest einen gegenüber dem Federrohr translatorisch gelagerten, bevorzugt gleitgelagerten, Schlitten umfasst. Bevorzugt umfasst die Federstange an einer ersten Seite einen gegenüber dem Federrohr translatorisch gelagerten ersten Schlitten und an einer zweiten Seite einen gegenüber dem Federrohr translatorisch gelagerten zweiten Schlitten umfasst.

Gemäß einer bevorzugten, also optionalen Ausführungsform ist das Federrohr an einer ersten Seite um eine erste Drehachse drehbar an einer ersten Befestigungseinheit angeordnet und ist die Federstange auf der ersten Seite um eine bevorzugt parallel zur ersten Drehachse orientierte zweite Drehachse drehbar mit einem Hebelelement verbunden. Das Hebelelement ist vorzugsweise wiederum auf seiner der Federstange gegenüberliegenden Seite - beabstandet von der ersten Drehachse - um eine zur ersten Drehachse bevorzugt parallel orientierte dritte Drehachse drehbar an der ersten Befestigungseinheit angeordnet. Entsprechend kann ein Federarmgetriebe bereitgestellt werden.

Zum Aufbringen einer Vorspannung auf die Federstange kann im Federrohr ein Federelement, bevorzugt eine Druckfeder, zum Vorspannen der Federstange relativ zum Federrohr vorgesehen sein, wobei sich das Federelement bevorzugt an einem Ende am Federrohr, bevorzugt an einem im Federrohr vorgesehenen Absatz, vorzugsweise umfassend eine Einbuchtung am Federrohr, abstützt, und an einem anderen Ende an der Federstange, bevorzugt an einem an der Federstange vorgesehenen Absatz, abstützt.

Gemäß einer bevorzugten Ausführungsform weisen die Kontaktfläche und/oder der Außenumfangsabschnitt zumindest teilweise eine - im Vergleich zu einer Materialpaarung der Grundmaterialien des die Kontaktfläche umfassenden Teils und des den Außenumfangsabschnitt umfassenden Teils - den Reibungskoeffizienten zwischen Kontaktfläche und Außenumfangsabschnitt erhöhende Beschichtung, bevorzugt eine Bremsbelagschicht, auf.

Die oben gestellte Aufgabe wird weiterhin durch ein Tragarmsystem zum Halten zumindest eines medizinischen Geräts mit den Merkmalen des Anspruchs 11 gelöst. Vorteilhafte Weiterbildungen ergeben sich aus der vorliegenden Beschreibung und den Figuren.

Entsprechend wird ein Tragarmsystem zum Halten zumindest eines medizinischen Geräts vorgeschlagen, welche zumindest eine Federarmvorrichtung gemäß einer der vorstehenden Ausführungsformen umfasst.

Die hinsichtlich der Federarmvorrichtung beschriebenen Vorteile und Wirkungen werden ebenfalls durch das Tragarmsystem erzielt, weshalb auf deren wiederholte Beschreibung an dieser Stelle verzichtet wird, um Redundanzen zu vermeiden.

### Kurze Beschreibung der Figuren

Bevorzugte weitere Ausführungsformen der Erfindung werden durch die nachfolgende Beschreibung der Figuren näher erläutert. Dabei zeigen:
Figur 1 schematisch ein Tragarmsystem mit einer Federarmvorrichtung;
Figur 2 schematisch eine perspektivische Seitenansicht eines Teilbereichs auf einer zweiten Seite der Federarmvorrichtung aus Figur 1;
Figur 3 schematisch eine perspektivische Schnittansicht durch die Federarmvorrichtung aus den Figuren 1 und 2 auf Höhe einer Klemmeinheit quer zu einer Längsrichtung; und
Figur 4 schematisch eine perspektivische Schnittansicht in Längsrichtung durch einen Teilbereich der Federarmvorrichtung aus den Figuren 1 bis 3.

### Detaillierte Beschreibung bevorzugter Ausführungsbeispiele

Im Folgenden werden bevorzugte Ausführungsbeispiele anhand der Figuren beschrieben. Dabei werden gleiche, ähnliche oder gleichwirkende Elemente in den unterschiedlichen Figuren mit identischen Bezugszeichen versehen, und auf eine wiederholte Beschreibung dieser Elemente wird teilweise verzichtet, um Redundanzen zu vermeiden.

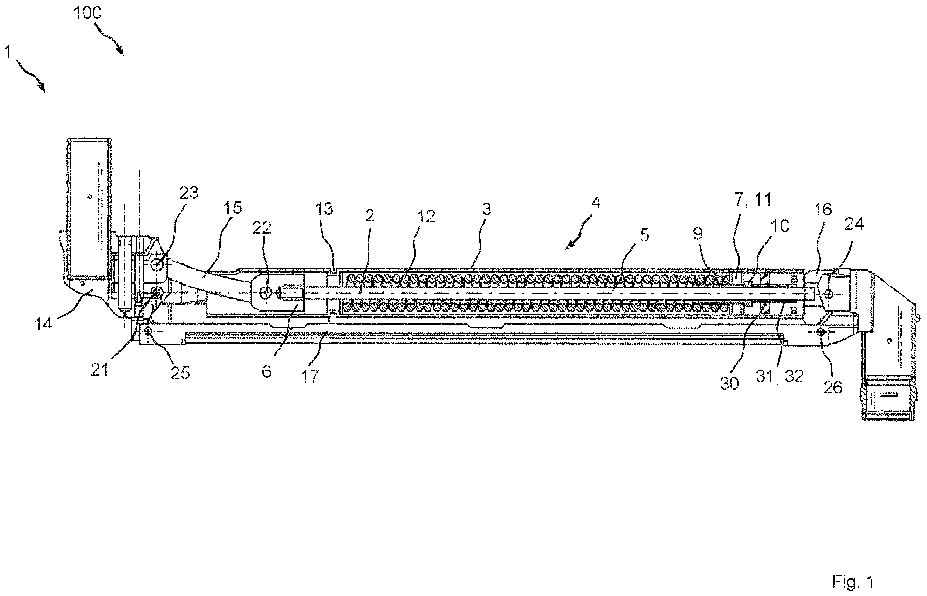

In Figur 1 wird schematisch ein Tragarmsystem 100 mit einer Federarmvorrichtung 1 gezeigt. Das Tragarmsystem 100 ist zum Halten eines medizinischen Geräts (nicht gezeigt) ausgebildet.

Die Federarmvorrichtung 1 umfasst ein sich in einer Längsrichtung 2 erstreckendes Federrohr 3. Im Inneren des Federrohrs 3 ist eine in Längsrichtung 2 relativ zum Federrohr 3 verschiebbare Federstange 4 angeordnet.

Die Federstange 4 weist eine sich in Längsrichtung 2 erstreckende Gewindestange 5 auf, welche an einer ersten Seite an einem gegenüber dem Federrohr 3 translatorisch gleitend gelagerten ersten Schlitten 6 befestigt, vorliegend eingeschraubt, ist, und an einer zweiten Seite mit einem gegenüber dem Federrohr 3 translatorisch gleitend gelagerten zweiten Schlitten 7 verbunden ist. Vorliegend umfasst der zweite Schlitten eine Gewindebuchse 9 mit Absatz 10, auf welchen ein Gleitring 11 aufgeschoben ist.

Der Gleitring 11 ist zugleich als Anschlag auf der zweiten Seite für ein Federelement 12, vorliegend ausgebildet als Druckfeder, zum Vorspannen der Federstange 4 relativ zum Federrohr 3 ausgebildet. Das Federelement 12 ist auf der ersten Seite an einer Einbuchtung 13 im Federrohr 3 abgestützt. Der Abstand in Längsrichtung 2 zwischen dem als erster Anschlag auf der zweiten Seite dienenden Gleitring 11 und der als zweiter Anschlag auf der ersten Seite dienenden Einbuchtung 13 ist kleiner als eine Nulllänge des Federelements 12, welches dieses in einem entspannten Zustand aufweist. Das Federelement 12 ist mithin vorgespannt. Die Höhe der Vorspannung des Federelements 12 kann durch ein Verändern der Position des zweiten Anschlags relativ zur Gewindestange 5 in Längsrichtung 2 betrachtet verändert werden. Hierzu kann die Gewindebuchse 9 relativ zur Gewindestange 5 gedreht werden. Gemäß der hier vorliegenden, also optionalen Ausführungsform der Federarmvorrichtung 1 ist die Verschraubung zwischen Gewindebuchse 9 und Gewindestange 5 selbsthemmend ausgebildet. Alternativ kann die Gewindebuchse auch über ein optionales Sicherungselement (nicht gezeigt) in ihrer Position relativ zur Gewindestange 5 gesichert sein.

Das Federrohr 3 ist an der ersten Seite um eine erste Drehachse 21 drehbar an einer ersten Befestigungseinheit 14 angeordnet. Ferner ist die Federstange 4 über ein Hebelelement 15 mit der ersten Befestigungseinheit 14 verbunden. Genauer ist die Federstange 4 auf der ersten Seite um eine parallel zur ersten Drehachse 21 orientierte zweite Drehachse 22 drehbar mit dem Hebelelement 15 verbunden, und das Hebelelement 15 ist auf seiner der Federstange 4 gegenüberliegenden Seite um eine von der ersten Drehachse 21 beabstandete, zur ersten Drehachse 21 parallel orientierte dritte Drehachse 23 drehbar an der ersten Befestigungseinheit 14 angeordnet.

Entsprechend stellt das vorgespannte Federelement 12 eine Vorspannung des Federrohres 3 bereit, wobei die Vorspannung einer auf das Federrohr 3 wirkenden Gewichtskraft entgegenwirkt.

Auf der zweiten Seite ist das Federrohr 3 über eine zur ersten Drehachse 21 parallel orientierte vierte Drehachse 24 drehbar an einer zweiten Befestigungseinheit 16 angeordnet.

Die Federarmvorrichtung 1 umfasst ferner eine Zug-Druckstange 17, welche über eine zur ersten Drehachse 21 parallel orientierte fünfte Drehachse 25 drehbar an der ersten Befestigungseinheit 14 angeordnet ist und über eine zur vierten Drehachse 24 parallel orientierte sechste Drehachse 26 drehbar an der zweiten Befestigungseinheit 16 angeordnet.

Entsprechend bildet die Federarmvorrichtung eine Gelenkvorrichtung aus, die eine Parallelführung der zweiten Befestigungseinheit 16 relativ zur ersten Befestigungseinheit 14 bereitstellt.

An der zweiten Befestigungseinheit 16 kann ein medizinisches Gerät (nicht gezeigt) oder eine weitere Federarmvorrichtung angebracht werden, wobei die zweite Befestigungseinheit 16 dann die erste Befestigungseinheit dieser weiteren Federarmvorrichtung darstellt.

Dadurch, dass das Hebelelement 15 und das Federrohr 3 voneinander beabstandet an dem ersten Befestigungselement oder -einheit 14 gelagert sind, ergibt sich ein je nach Winkelstellung des Federrohres 3 um die erste Drehachse 21 unterschiedlicher Hebelarm bzw. Winkel, mittels welches das Hebelelement 15 das Federrohr 3 über die Federstange 4 und das Federelement 12 abstützt. So kann bei unterschiedlichen Stellungen der Federarmvorrichtung 1 bzw. des Federrohres 3 eine ähnliche oder gar im Wesentlichen die gleiche Gewichtskraft gleichermaßen abgestützt werden.

Der Verbund aus Federrohr 3, Federarm 4 und Federelement 12 kann als balancierter Federarm verstanden werden.

Weitere Details zur Ausbildung der Federarmvorrichtung bzw. des Federarms können der WO 2016/074787 A1, darin insbesondere Seite 14, Zeile 14 bis Seite 20, Zeile 20, und der EP 3 861 958 A1, darin insbesondere Absätze 48 bis 59 entnommen werden, auf deren Inhalt an dieser Stelle erneut verwiesen sei.

Die Federarmvorrichtung 1 umfasst ferner eine Klemmeinheit 30 zum lösbaren Aufbringen einer Klemmkraft auf die Federstange 4. Die Klemmeinheit 30 ist gemäß dieser optionalen Ausführungsform im Bereich der zweiten Seite in Längsrichtung 2 betrachtet ortsfest am Federrohr 3 angeordnet.

Die Federstange 4 umfasst eine auf die Gewindestange 5 aufgeschraubte, zylinderförmige Hülse 31, die einen Außenumfangsabschnitt 32 bereitstellt.

Gemäß dieser optionalen Ausführungsform ist die Hülse 31 drehfest an der Gewindestange 5 via Selbsthemmung angeordnet. Alternativ oder zusätzlich kann die Hülse 31 auch mittels eines Sicherungselements (nicht gezeigt), etwa einem Sicherungsring, einem Sicherungsstift oder einer Sicherungsschraube, an der Gewindestange 5 in ihrer Position fixiert sein. Auch kann die Hülse 31 auf die Gewindestange 5 aufgepresst sein und/oder die Gewindestange 5 und die Hülse 31 in einem verspannten Zustand verschraubt sein, beispielsweise, indem die Hülse 31 gegen die Gewindebuchse 9 gekontert ist.

Die Klemmeinheit 30 ist derart ausgebildet, dass durch das Aufbringen der Klemmkraft auf die Federstange 4 die Federstange 4 in ihrer Position relativ zum Federrohr 3 arretiert ist.

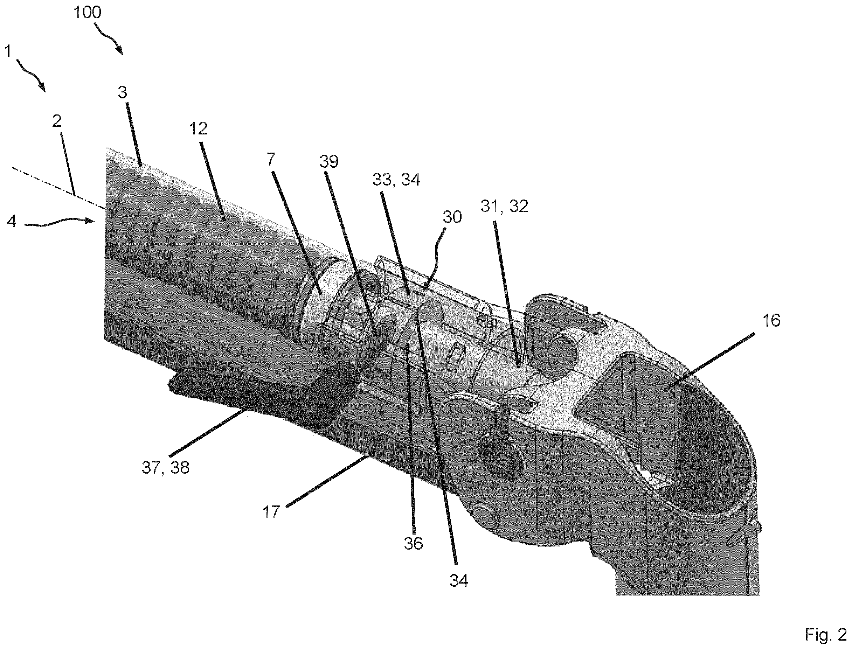

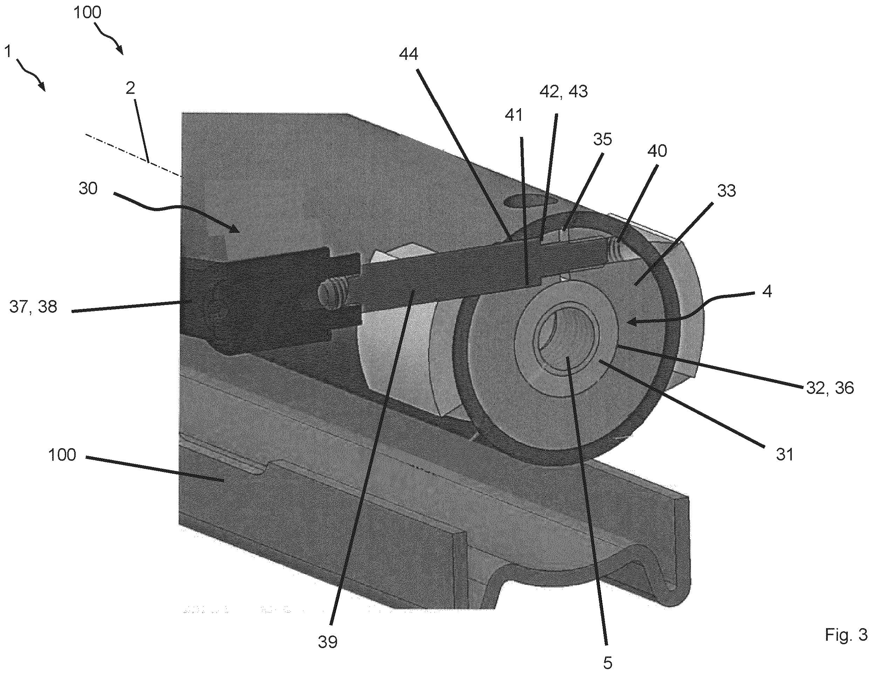

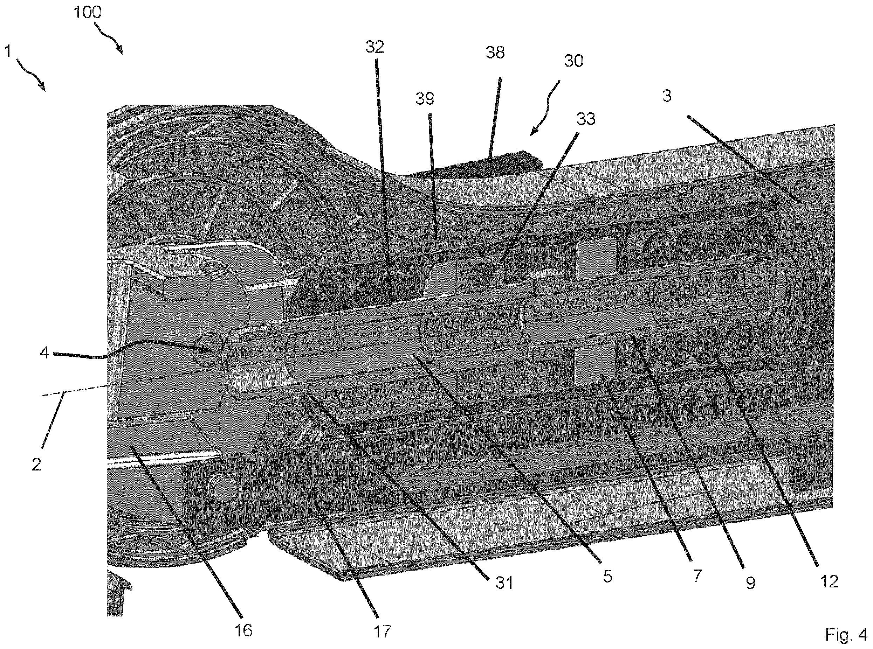

Vorteilhafte Details der Klemmeinheit 30 werden im Folgenden mit Bezug auf die Figuren 2 bis 4 näher beschrieben. Figur 2 zeigt schematisch eine perspektivische Seitenansicht eines Teilbereichs auf der zweiten Seite der Federarmvorrichtung 1 aus Figur 1, wobei das Federrohr 3 teiltransparent dargestellt ist, um die darin aufgenommenen Bauteile erkennen zu können. Aus Figur 3 ist schematisch eine perspektivische Schnittansicht durch die Federarmvorrichtung 1 aus den Figuren 1 und 2 auf Höhe der Klemmeinheit 30 zu entnehmen. Figur 4 zeigt schematisch eine perspektivische Schnittansicht durch einen Teilbereich der Federarmvorrichtung 1 aus den Figuren 1 bis 3 entlang der Längsrichtung 2.

Vorliegend umfasst die Klemmeinheit 30 einen Klemmring 33, der im Inneren des Federrohres 3 eingeschoben ist. Der Klemmring 33 ist vorliegend aus einem ringförmigen Grundkörper 34 mit einem sich radial durch den Grundkörper 34 erstreckenden Schlitz 35 ausgebildet. Der Grundköper 34 ist also ein Ringsegment mit sich am Schlitz 35 gegenüberliegenden freien Enden, wobei die gegenüberliegenden freien Enden in einem unbelasteten Zustand des Klemmrings 33 mit einem vorgegebenen Abstand (der Breite des Schlitzes 35) voneinander beabstandet sind.

Werden die freien Enden des Klemmrings 33 aus dem unverspannten Zustand aufeinander zu gedrückt, so verringert sich entsprechend der Teilkreisdurchmesser, auf welcher die radiale Innenfläche vorliegt. Die radiale Innenfläche des Klemmrings 33 stellt eine Kontaktfläche 36 zum Aufbringen der Klemmkraft auf den Außenumfangsabschnitt 32 der Federstange 4 dar. Bei einem Zusammendrücken des Klemmrings 33 kann folglich die durch den Klemmring 33 geführte Federstange 4 geklemmt werden.

Der Klemmring 33 kann über eine vorliegend in Form eines Klemmhebels 37 (siehe Figuren 2 und 3) ausgebildete Betätigungseinheit zwischen einem gelösten Zustand und einem die Klemmkraft aufbringenden Zustand gewechselt werden. Im gelösten Zustand ist ein Gleiten des Außenumfangsabschnitts 32 relativ zur Kontaktfläche 36 und somit ein Verschieben der Federstange 4 relativ zum Federrohr 3 ermöglicht. Vorliegend ist im gelösten Zustand der durch die Kontaktfläche 36 definierte Innenradius des Klemmrings 33 größer als der Außenradius des Außenumfangsabschnitts 32.

Der Klemmhebel 37 umfasst vorliegend einen Gewindebolzen 39 und einen an dessen einem Ende befestigten Hebelgriff 38. Am anderen Ende ist der Gewindebolzen 39 in ein im Klemmring 33 ausgebildeten Gewinde 40 eingeschraubt. In Bezug auf den Spalt oder Schlitz 35 gegenüberliegend ist der Gewindebolzen 39 in einer Bohrung 41 im Klemmring 33 geführt und stützt sich mit einem Bolzenabsatz 42 an einem Absatz 43 in der Bohrung 41 in axialer Richtung des Gewindebolzens 39 ab.

Der Gewindebolzen 39 ist ferner in einer im Federrohr 3 vorgesehenen Aufnahmebohrung 44 durch das Federrohr 3 geführt. Dadurch ist der Gewindebolzen 39 in seiner Position relativ zum Federrohr 3 fixiert und lediglich in axialer Richtung der Aufnahmebohrung 44 verschiebbar und in dieser um die axiale Richtung der Aufnahmebohrung 44 drehbar in dieser gehalten. Da der Gewindebolzen 39 ebenfalls durch die Bohrung 41 verläuft, ist ebenfalls der Klemmring 33 und somit die Klemmeinheit 30 in Bezug auf die Längsrichtung 2 relativ zum Federrohr 3 ortsfest gehalten.

Durch Einschrauben des Gewindebolzes 39 via dem Klemmhebel 37 tiefer in das Gewinde 40 erfolgt eine Verkleinerung des Teilkreisdurchmessers der Kontaktfläche 36, so dass die Klemmkraft auf die Federstange 4, genauer auf die Hülse 31, aufgebracht werden kann. Durch Herausschrauben des Gewindebolzens 39 via dem Klemmhebel 37 kann die Klemmkraft wieder gelöst werden.

Durch Einstellen des Klemmhebels 37 in eine vorgegebene Winkelposition um die Längsachse des Gewindebolzens 39, welche optional durch eine Markierung an der Außenseite eines hier nicht gezeigten Gehäuseteiles der Federrohrvorrichtung 1 angezeigt sein kann, kann die Anpresskraft zwischen Kontaktfläche 36 und Außenumfangsabschnitt 32 eine vorgegebene Höhe aufweisen.

Die Kontaktfläche 36 und/oder der Außenumfangsabschnitt 32 können optional zumindest teilweise eine bevorzugt den Reibungskoeffizienten zwischen Kontaktfläche 36 und Außenumfangsabschnitt 32 erhöhende Beschichtung, bevorzugt eine Bremsbelagschicht, aufweisen.

Der maximale Bewegungsumfang der Federstange 4 relativ zum Federrohr 3, bei welchem die Klemmeinheit 30 die Klemmkraft auf die Federstange 4 ausüben kann, ist durch die Länge in Längsrichtung 2 des Außenumfangabschnitts 32 vorgegeben, welche in Figur 4 zu erkennen ist.

Soweit anwendbar, können alle einzelnen Merkmale, die in den Ausführungsbeispielen dargestellt sind, miteinander kombiniert und/oder ausgetauscht werden, ohne den Bereich der Erfindung zu verlassen.

### Bezugszeichenliste

- 1: Federarmvorrichtung
- 2: Längsrichtung
- 3: Federrohr
- 4: Federstange
- 5: Gewindestange
- 6: Erster Schlitten
- 7: Zweiter Schlitten
- 9: Gewindebuchse
- 10: Absatz
- 11: Gleitring
- 12: Federelement
- 13: Einbuchtung
- 14: Erste Befestigungseinheit
- 15: Hebelelement
- 16: Zweite Befestigungseinheit
- 17: Zug-Druckstange
- 21: Erste Drehachse
- 22: Zweite Drehachse
- 23: Dritte Drehachse
- 24: Vierte Drehachse
- 25: Fünfte Drehachse
- 26: Sechste Drehachse
- 30: Klemmeinheit
- 31: Hülse
- 32: Außenumfangsabschnitt
- 33: Klemmring
- 34: Grundkörper
- 35: Schlitz
- 36: Kontaktfläche
- 37: Klemmhebel
- 38: Hebelgriff
- 39: Gewindebolzen
- 40: Gewinde
- 41: Bohrung
- 42: Bolzenabsatz
- 43: Absatz
- 44: Aufnahmebohrung
- 100: Tragarmsystem

## Patentansprüche

1. Federarmvorrichtung (1) für ein Tragarmsystem (100) zum Halten eines medizinischen Geräts, umfassend ein Federrohr (3) und eine zumindest teilweise im Inneren des Federrohres (3) angeordnete, in Längsrichtung (2) des Federrohres (3) relativ zum Federrohr (3) verschiebbare Federstange (4),
**dadurch gekennzeichnet, dass**
eine Klemmeinheit (30) zum lösbaren Aufbringen einer Klemmkraft auf die Federstange (4) am Federrohr (3) in Längsrichtung (2) betrachtet ortsfest angeordnet ist.

2. Federarmvorrichtung (1) gemäß Anspruch 1, **dadurch gekennzeichnet, dass** das die Klemmeinheit (30) derart ausgebildet ist, dass durch das Aufbringen der Klemmkraft auf die Federstange (4) die Federstange (4) in ihrer Position relativ zum Federrohr (3) arretiert ist.

3. Federarmvorrichtung (1) gemäß Anspruch 1 oder 2, **dadurch gekennzeichnet, dass** die Klemmeinheit (30) eine Kontaktfläche (36) zum Aufbringen der Klemmkraft auf einen Außenumfangsabschnitt (32) der Federstange (4) aufweist.

4. Federarmvorrichtung (1) gemäß dem vorstehenden Anspruch, **dadurch gekennzeichnet, dass** die Klemmeinheit (30) derart ausgebildet ist, dass im Zustand des Aufbringens der Klemmkraft die Kontaktfläche (36) mit einer vorgegebenen Anpresskraft an den Außenumfangsabschnitt (32) gepresst ist.

5. Federarmvorrichtung (1) gemäß einem der vorstehenden Ansprüche, **dadurch gekennzeichnet, dass** die Klemmeinheit (30) zumindest ein Klemmstück, bevorzugt zumindest eine Klemmbacke, und/oder einen Klemmring (33) umfasst, wobei das zumindest eine Klemmstück und/oder der Klemmring (33) die Kontaktfläche (36) umfasst.

6. Federarmvorrichtung (1) gemäß einem der vorstehenden Ansprüche, **dadurch gekennzeichnet, dass** die Klemmeinheit (30) eine Betätigungseinheit zum Aufbringen und Lösen der Klemmkraft umfasst.

7. Federarmvorrichtung (1) gemäß dem vorstehenden Anspruch, **dadurch gekennzeichnet, dass** die Betätigungseinheit einen Klemmhebel (37) oder einen Exzenterhebel umfasst, und/oder die Betätigungseinheit einen manuell betätigbaren Griff, bevorzugt einen Hebelgriff (38), einen Schraubgriff oder einen Sterngriff, umfasst.

8. Federarmvorrichtung (1) gemäß einem der vorstehenden Ansprüche, **dadurch gekennzeichnet**, die Federstange (4) eine Gewindestange (5) umfasst.

9. Federarmvorrichtung (1) gemäß dem vorstehenden Anspruch, **dadurch gekennzeichnet, dass** die Gewindestange (5) einen den Außenumfangsabschnitt (32) bereitstellenden Zylinderflächenabschnitt aufweist und/oder eine auf ein Gewinde der Gewindestange (5) aufgeschraubte, den Außenumfangsabschnitt (32) bereitstellenden Hülse (31) umfasst.

10. Federarmvorrichtung (1) gemäß dem vorstehenden Anspruch, **dadurch gekennzeichnet, dass** die Hülse (31) drehfest an der Gewindestange (5) angeordnet ist.

11. Federarmvorrichtung (1) gemäß dem vorstehenden Anspruch, **dadurch gekennzeichnet, dass** die Hülse (31) mittels eines Sicherungselements, einem Sicherungsring, einem Sicherungsstift oder einer Sicherungsschraube an der Gewindestange (5) in ihrer Position relativ zur Gewindestange (5) fixiert ist, und/oder die Hülse (31) auf die Gewindestange (5) aufgepresst ist, und/oder die Gewindestange (5) und die Hülse (31) in einem verspannten Zustand verschraubt vorliegen, und/oder die Verschraubung von Gewindestange (5) und Hülse (31) selbsthemmend ausgebildet ist.

12. Federarmvorrichtung (1) gemäß einem der vorstehenden Ansprüche, **dadurch gekennzeichnet**, die Federstange (4) im Federrohr (3) translatorisch gelagert, bevorzugt gleitgelagert, ist, wobei bevorzugt die Federstange (4) zumindest einen gegenüber dem Federrohr (3) translatorisch gelagerten, bevorzugt gleitgelagerten, Schlitten (6, 7) umfasst, wobei bevorzugt die Federstange(4) an einer ersten Seite einen gegenüber dem Federrohr (3) translatorisch gelagerten ersten Schlitten (6) umfasst und an einer zweiten Seite einen gegenüber dem Federrohr (3) translatorisch gelagerten zweiten Schlitten (7) umfasst.

13. Federarmvorrichtung (1) gemäß einem der vorstehenden Ansprüche, **dadurch gekennzeichnet, dass** das Federrohr (3) an einer ersten Seite um eine erste Drehachse (21) drehbar an einer ersten Befestigungseinheit (14) angeordnet ist und die Federstange (4) auf der ersten Seite um eine parallel zur ersten Drehachse (21) orientierte zweite Drehachse (22) drehbar mit eine Hebelelement (15) verbunden ist, und das Hebelelement (15) auf seiner der Federstange (4) gegenüberliegenden Seite um eine von der ersten Drehachse (21) beabstandete, zur ersten Drehachse (21) parallel orientierte dritte Drehachse (23) drehbar an der ersten Befestigungseinheit (14) angeordnet ist.

14. Federarmvorrichtung (1) gemäß einem der vorstehenden Ansprüche, **dadurch gekennzeichnet, dass** im Federrohr (3) ein Federelement (12), bevorzugt eine Druckfeder, zum Vorspannen der Federstange (4) relativ zum Federrohr (3) vorgesehen ist, wobei sich das Federelement (12) bevorzugt an einem Ende am Federrohr (3), bevorzugt an einem im Federrohr (3) vorgesehene Absatz, abstützt und am anderen Ende an der Federstange (4), bevorzugt an einem an der Federstange (4) vorgesehene Absatz, abstützt.

15. Federarmvorrichtung (1) gemäß einem der vorstehenden Ansprüche, **dadurch gekennzeichnet, dass** die Kontaktfläche (36) und/oder der Außenumfangsabschnitt (32) zumindest teilweise eine den Reibungskoeffizienten zwischen Kontaktfläche (36) und Außenumfangsabschnitt (32) erhöhende Beschichtung, bevorzugt eine Bremsbelagschicht, aufweisen.

16. Tragarmsystem (100) zum Halten zumindest eines medizinischen Geräts, umfassend zumindest eine Federarmvorrichtung (1) gemäß einem der vorstehenden Ansprüche.
